# FASCICULE DE BREVET EUROPEEN

(11) **EP 3 700 648 B1**
(45) Date de publication et mention de la délivrance du brevet: **08.12.2021**
(21) Numéro de dépôt: 18785405.4
(22) Date de dépôt: 17.10.2018
(51) Int. Cl.: B01D 15/18

(54) **NOUVEAU SYSTÈME DE DISTRIBUTION PAR PANNEAUX MÉRIDIENS POUR UN PROCÉDÉ DE SÉPARATION EN LIT MOBILE SIMULÉ UTILISANT N-COLONNES EN SÉRIE**
NEUARTIGES VERTEILUNGSSYSTEM MIT VERWENDUNG VON MERIDIANPANEELEN FÜR EIN SIMULIERTES WANDERBETTTRENNVERFAHREN UNTER VERWENDUNG VON N-SPALTEN IN SERIE
NOVEL DISTRIBUTION SYSTEM USING MERIDIAN PANELS FOR A SIMULATED MOVING BED SEPARATION METHOD USING N COLUMNS IN SERIES

(30) Priorité: 27.10.2017 FR 1760168
(43) Date de publication de la demande: 02.09.2020
(73) Titulaire: IFP Energies nouvelles, 92852 Rueil-Malmaison Cedex (FR)
(72) Inventeur: ROYON-LEBEAUD, Aude, 69007 Lyon (FR); AUGIER, Frédéric, 69360 Saint-Symphorien-d'Ozon (FR)
(86) Numéro de dépôt international: PCT/EP2018/078434
(87) Numéro de publication internationale: WO 2019/081311

(56) Documents cités:
- EP-A1- 1 913 988
- CN-A- 103 285 621
- FR-A1- 2 930 454
- US-A1- 2014 183 281

## Description

### CONTEXTE DE L'INVENTION

La présente invention concerne un dispositif d'introduction et de collecte des fluides dans le procédé de séparation des xylènes en lit mobile simulé (noté en abrégé LMS), et les unités mettant en œuvre ledit procédé, plus particulièrement les unités de grands diamètres (D > 4m), et possédant de nombreux étages de séparation avec injection ou soutirage de produits entre deux étages.

Le dispositif selon l'invention a la particularité de respecter un temps de séjour à peu près égal pour l'ensemble des particules fluide entrant dans le canal de distribution, traversant le lit, et étant évacué par un canal de collecte, symétrique du canal de distribution.

### EXAMEN DE L'ART ANTERIEUR

Les technologies actuelles de séparation en lit mobile simulé (en abrégé LMS dans la suite du texte) utilisent des unités qui comportent un certain nombre de points communs :
- une succession de lits d'adsorbant au sein desquels s'écoule le débit de « tourne en rond ». Ce débit de tourne en rond représente en général plusieurs fois le débit de charge entrant (environ entre 1,5 et 6 fois). On l'appelle dans la terminologie anglo saxonne « pump around ».
- des systèmes d'injection de la charge et du solvant, et de soutirage des effluents appelés extrait et raffinat,
- des systèmes de collecte et redistribution pour passer d'un lit au lit suivant.

Dans les procédés de séparation par adsorption en lit mobile simulé on dispose généralement d'une pluralité de lits localisés dans une ou deux colonnes d'adsorption. Entre chaque lit se situent des panneaux distributeurs-mélangeurs-extracteurs ou « DME » alimentés par des lignes qui présentent le plus souvent la forme d'« araignées de distribution/ extraction ». Chaque panneau DME situé entre deux lits consécutifs est relié à l'extérieur au moyen d'une ou deux lignes ou réseaux aboutissant à une vanne mettant en communication successivement chacun des lits avec chacun des flux entrant dans ou sortant de la section d'adsorption. Cette opération est réalisée de manière séquentielle, et le temps au bout duquel on revient au lit de départ s'appelle le temps de cycle qui est une donnée importante du procédé.

Par exemple, le brevet US2985589 montre clairement que chacun des réseaux d'injection ou soutirage est relié par une seule ligne à une vanne qui connecte successivement la charge, l'extrait, le solvant puis le raffinat. Cette manière de procéder présente l'inconvénient de diminuer considérablement les performances du procédé puisque chaque flux se trouve ainsi contaminé par le contenu de la ligne commune. Il est donc indispensable de mettre en place un système de rinçage.

Plusieurs brevets expliquent comment mettre en œuvre ces rinçages, notamment les brevets FR275188, FR2772634, FR2870751.

Les rinçages s'avèrent en général coûteux en terme d'investissement (vanne, ligne supplémentaires), aussi bien qu'en terme de cout opératoire (rendement, productivité).

Les « araignées de distribution/extraction » constituent des obstacles au sein du lit d'adsorbant qui perturbent l'écoulement dans le lit. Le brevet WO2009133254 montre comment minimiser l'impact des obstacles sur l'hydrodynamique dans le lit.

L'article Augier et al 2008 (Séparation and Purification Technology 63, pp466-474) évalue le coût des obstacles.

### DESCRIPTION SOMMAIRE DES FIGURES

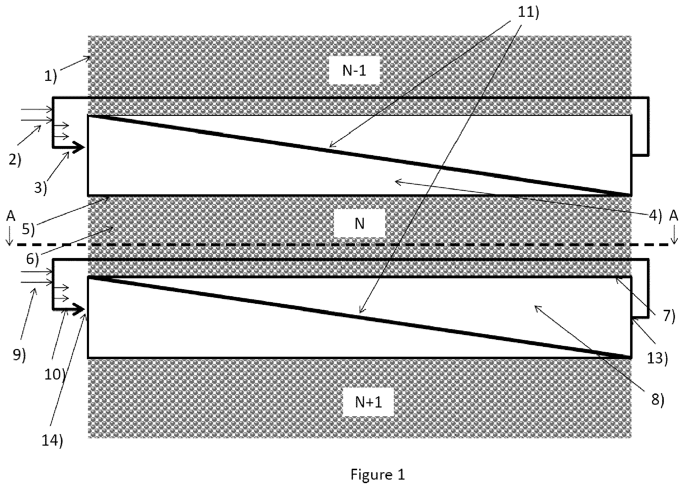

La figure 1 montre une vue de profil de 3 plateaux successifs, notés N-1 ; N et N+1 de haut en bas. Elle permet de bien visualiser le canal de distribution (4) et le canal de collecte (8), symétrique l'un de l'autre et séparés par la paroi (11), avec le retour du fluide depuis la sortie (7) du canal de collecte (8) jusqu'à l'entrée (10) du canal de distribution dans le lit N+1.

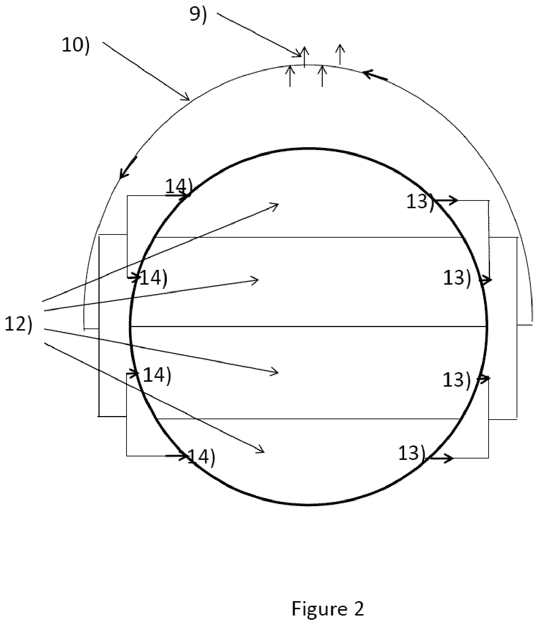

La figure 2 correspond à une coupe selon la ligne A-A de la figure 1. Elle permet donc de visualiser le plateau et sa division en panneaux méridiens ainsi que la collecte des fluides par les éléments (13) et leur distribution par les éléments (14). La collecte par les éléments (13) est regroupée en deux flux, puis encore regroupée s'il le faut. De la même manière, la distribution par les éléments (14) peut s'effectuer en plusieurs divisions en deux du flux principal provenant de la conduite (10).

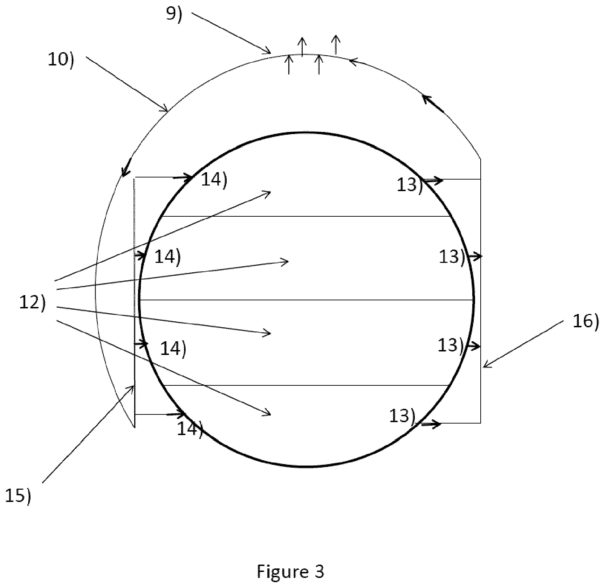

La figure 3 montre une variante du système de distribution/collecte dans laquelle l'ensemble des flux collectés par les conduites (13) sur les différents panneaux est repris sous forme de clarinette en une seule conduite (10) avant d'être redistribué sous forme de clarinette dans chaque panneau par les conduites (14).

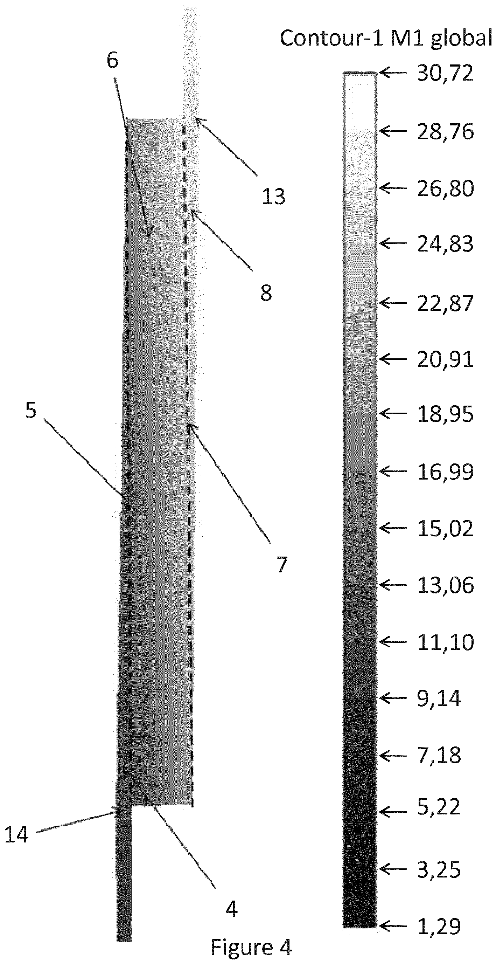

La figure 4 est une visualisation résultant d'une simulation numérique. Il s'agit d'une vue en coupe d'un panneau, selon un plan de coupe identique à celui de la figure 1. L'entrée dans le canal de distribution se fait par le bord en haut à gauche. Le lit d'adsorbant est la zone comprise entre les deux grilles schématisées par une ligne pointillée. La sortie du canal de collecte se fait par le bord en bas à droite. Le champ de niveaux de grisés se réfère à l'âge interne moyen du fluide dans le lit (M1) en secondes.

### DESCRIPTION SOMMAIRE DE L'INVENTION

La présente invention peut se définir comme un système/dispositif de distribution et de collecte pour les unités de séparation en lit mobile simulé de diamètre supérieur à 4 mètres, préférentiellement supérieure à 7 mètres, l'unité comportant au moins une colonne de séparation divisé en N lits d'adsorbant supporté par le plateau N, chaque plateau N étant lui-même divisé en panneaux méridiens, c'est-à-dire en panneaux parallèles entre eux et contigus de manière à assurer une couverture complète de la section dudit lit, et chaque panneau étant alimenté par un canal de distribution (4).

Le soutirage des effluents dudit panneau se fait par un canal de collecte (8), le canal de distribution et le canal de collecte entourant chaque panneau ayant des hauteurs variant linéairement sur toute la longueur du panneau, et étant telles que la vitesse d'entrée dans le lit de chaque tranche de fluide reste la même depuis la section d'entrée du panneau jusqu'à sa section de sortie du panneau, et que la somme des hauteurs du canal de distribution et du canal de collecte prise en tout point de la longueur du panneau reste constante. Pour la bonne clarté des différentes dimensions des canaux, on appelle longueur la dimension du canal correspondant à la distance séparant l'entrée du canal de sa sortie, largeur du canal la dimension horizontale perpendiculaire à la longueur, et hauteur la dimension verticale perpendiculaire à la longueur.

Plus précisément, la hauteur du canal de distribution décroit linéairement depuis l'entrée jusqu'à la sortie, et la hauteur du canal de collecte augmente linéairement depuis l'entrée jusqu'à la sortie.

A chaque abscisse x correspondant à un point courant M de la longueur du panneau , la somme des hauteurs du canal de distribution et du canal de collecte est constante.

Le système de distribution et de collecte selon l'invention utilise une conduite périphérique (10) extérieure à la colonne qui permet de relier les différents canaux de collecte du plateau N aux différents plateaux de distribution du plateau N+1, ladite conduite permettant d'effectuer les injections de charge et de solvant et les soutirages de raffinat et d'extrait.

Dans une première variante du système de distribution et de collecte pour les unités de séparation en LMS selon l'invention (représentée figure 2), la distribution des fluides sur les différents panneaux d'un lit N se fait par divisions successives par deux du flux provenant de la conduite (10) pour alimenter les entrées (14) de deux panneaux adjacents, et la collecte des effluents du lit N se fait également par rassemblement deux à deux des sorties (13) de deux panneaux adjacents, qui alimente la conduite (10) du lit N+1.

Dans une seconde variante du système de distribution et de collecte pour les unités de séparation en LMS selon l'invention (représentée figure 3), la distribution des fluides sur les différents panneaux d'un plateau N se fait à partir d'une clarinette de distribution (15) qui alimente directement les différentes entrées (14) de chaque panneau, et la collecte des effluents dudit lit N se fait de la même manière directement au moyen d'une clarinette de collecte (16) qui récupère les effluents des sorties (13) de chaque panneau.

La présente invention porte également sur un procédé utilisant le dispositif de distribution et de collecte selon l'invention, procédé dans lequel le fluide entrant au niveau d'un plateau N est introduit dans chaque panneau dudit plateau N par l'intermédiaire de conduites d'entrée (14), chaque entrée (14) alimentant un canal de distribution (4), dont la hauteur est maximale en entrée et minimale en sortie dudit canal, chaque tranche de fluide alimentant une fraction du lit granulaire situé immédiatement au-dessous du canal de distribution, et ladite fraction de fluide quittant le lit granulaire pour entrer dans le canal de collecte (8) situé immédiatement au-dessous du lit granulaire, le dit canal de collecte ayant sa hauteur maximale en sortie et sa hauteur minimale en entrée, l'effluent de sortie étant alors récupéré par la conduite périphérique extérieure (10), et étant réintroduit par les entrées (14) du lit N+1 dans le canal de distribution dudit lit N+1.

Le présent dispositif s'applique particulièrement au procédé de séparation des xylènes en lit mobile simulé fonctionnant avec un nombre de lits compris entre 4 et 24 et préférentiellement compris entre 8 et 12.

### DESCRIPTION DETAILLEE DE L'INVENTION

La présente invention consiste en un dispositif applicable aux unités en lit mobile simulé, dispositif permettant :
- d'assurer une collecte globale du flux de « pump around » pour s'affranchir des rinçages,
- de minimiser les obstacles au sein du lit. Le « pump around » est la terminologie anglo saxonne utilisée par l'homme du métier pour désigner le débit circulant dans l'ensemble de la colonne.

La collecte globale du flux de « pump around » est un enjeu extrêmement important sur les unités en lit mobile simulé, puisqu'elle permet d'éliminer les rinçages.

La technologie décrite dans la présente invention utilise le principe de compensation des temps de séjour au sein des zones de collecte et de distribution pour minimiser les variances, c'est-à-dire les écarts de temps de séjour des fluides circulant dans l'unité en fonction du point de départ et du point d'arrivée desdits fluides.

De plus les volumes inter-lits sont minimisés en travaillant à iso-vitesse plutôt qu'à iso-hauteur de canal dans les zones de collecte et distribution. L'encombrement total de la colonne est également minimisé en empilant les lits. Il n'y a donc pas de gestion de volume inter-lit spécifique.

Le débit provenant du lit N-1 (1) est collecté dans la conduite (3).

Les injections ou soutirages sont réalisés par le réseau de conduites (2).

Le canal de distribution (4) assure une distribution uniforme du débit dans le lit N, noté (6) sur la figure 1, au travers de la grille (5).

Le débit est collecté par le canal de collecte (8) au travers de la grille inférieure (7).

L'ensemble du débit est rassemblé dans la conduite (10) pour être réinjecté au lit N+1, situé immédiatement au-dessous du lit N, après injection ou soutirage par le réseau (9).

Les plaques de séparation (11) séparent le canal de collecte du canal de distribution (4). Il est important d'assurer la planéité de la plaque de séparation (11) par tout moyen connu de l'homme du métier. On pourra par exemple rendre la plaque de séparation (11) solidaire des grilles inférieure et supérieure (5) et (7).

On peut également utiliser des tirants s'étendant sur toute la largeur du panneau, rapportés sur des poutres ou plaques qui viennent délimiter lesdits panneaux sur la hauteur des canaux de collecte et distribution.

La hauteur à l'entrée du canal de distribution (4) est définie par une vitesse débitante maximale admissible de manière à ne pas déséquilibrer l'alimentation du lit. Typiquement, la vitesse débitante maximale admissible est comprise entre 0,1 et 5 m/s, idéalement entre 0,5 et 2,5 m/s.

La section de canal de distribution (4), diminue linéairement pour garantir une vitesse quasiment uniforme sur toute la longueur du canal, égale à cette vitesse débitante maximale. Cette constance de la vitesse provient du fait que le débit de fluide est toujours proportionnel à la section entrante, ceci sur chaque section entrante du canal.

Le profil de hauteur du canal de distribution est donc linéaire pour assurer cette proportionnalité.

Les canaux de collecte (8) et distribution (4) sont complémentaires au sens où le canal de distribution (4) situé immédiatement au-dessus du lit N est associé au canal de collecte (8) situé immédiatement au-dessous du dit lit N. Le flux quittant le canal de collecte (8) est ensuite dirigé vers le canal de distribution du lit N+1 au moyen de la conduite (10) visible sur la figure 2.

Cette conduite (10) suit grossièrement le périmètre cylindrique de l'unité pour se retrouver en position d'entrer dans le canal de distribution du lit N+1. Le réseau est dimensionné pour que la vitesse maximale débitante ne dépasse pas une certaine vitesse maximale, générale prise entre 4 et 6 m/s (pour des raisons de vibration).

Les distributions et collectes sont organisées en panneaux méridiens (12). On entend par panneaux méridiens le fait que les panneaux sont parallèles les uns aux autres et contigus, de manière à assurer une couverture totale de la section de l'unité. Le nombre de panneaux couvrant une section de l'unité varie entre 2 et 12, préférentiellement entre 4 et 8.

Les plateaux sont préférentiellement organisés en panneaux de section constante. Les débits sont ajustés pour avoir la même vitesse au sein du lit.

Le réseau externe de conduites (10) est également conçu pour fonctionner à iso temps de séjour aussi bien en configuration 1 (figure 2), qu'en configuration 2 (figure 3).

La configuration 1 correspond à un regroupement par deux des flux sortant de chaque panneaux.

La configuration 2 correspond à un regroupement direct de l'ensemble des flux sortant de chaque panneau.

Les deux configurations sont représentées sur les figures 2 et 3 qui correspondent à la coupe selon A-A de la figure 1.

On parle de temps de séjour « réseau » pour désigner le temps de séjour d'une particule fluide depuis son point de sortie de la colonne, pris au niveau de son point d'entrée dans la colonne et ce pour chacun des panneaux.

On peut distinguer :
- le temps de séjour réseau côté collecte, qui est le temps de séjour de la particule fluide depuis son point de sortie (13) de la colonne depuis n'importe quel panneau, jusqu'aux points d'injection et soutirage (9),
- le temps de séjour réseau côté distribution qui est le temps de séjour de la particule fluide depuis le point d'injection et soutirage (9) jusqu'à son point d'entrée (14) dans la colonne vers n'importe quel panneau.

Dans la configuration 1 le réseau (10) est organisé de telle manière que toutes les particules fluide ont :
- un temps de séjour réseau côté collecte identique, au sein du réseau des points de sortie de chaque panneau (13) au point d'injection ou soutirage global (9). C'est-à-dire que chaque particule fluide qui quitte la colonne depuis n'importe quel panneau met le même temps à parcourir la distance au sein du réseau depuis son point de sortie (13) de la colonne jusqu'au point d'injection/ soutirage (9).
- un temps de séjour réseau côté distribution identique au sein du réseau depuis le point d'injection ou soutirage global (9) jusqu'aux points d'entrée de chaque panneau (14). C'est-à-dire que chaque particule fluide met le même temps à parcourir la distance au sein du réseau depuis le point d'injection/ soutirage (9) jusqu'à son point d'entrée (14) dans la colonne vers n'importe quel panneau.

Les sorties de chaque panneau (13) et les entrées de chaque panneau (14) peuvent être réalisées au moyen de 1 à 6 points de sortie (respectivement d'entrée).

Le réseau externe de conduites (10) peut être à temps de séjour compensé entre zone de collecte et zone de distribution, comme montré sur la figure 3 qui représente la coupe A-A de la figure 1 dans la configuration 2.

Dans la configuration 2, les temps de séjour réseau ne sont pas identiques séparément entre temps de séjour réseau côté collecte et temps de séjour réseau côté distribution. En d'autres termes, dans la configuration 2, le fluide est désynchronisé entre les panneaux, en plus de la désynchronisation de temps de séjour inhérente à chaque panneau. La désynchronisation des temps de séjour à l'entrée des différents panneaux n'a pas d'impact sur les performances, car elle est compensée par une désynchronisation opposée opérée par le réseau de collecte, ceci en raison de la géométrie inversée des canaux d'alimentation et de collecte.

Le réseau externe (10) est organisé de telle manière que toutes les particules fluide ont un temps de séjour identique depuis le point de sortie du panneau (13) jusqu'au point d'entrée du panneau (14), mais réparti différemment suivant le panneau entre temps de séjour avant le point d'injection ou soutirage global (9) - temps de séjour réseau côté collecte - et temps de séjour après le point d'injection ou soutirage global (9) jusqu'au point d'entrée dans la colonne (14) - temps de séjour réseau côté distribution.

Les entrées de chaque panneau (14) peuvent être réalisées par l'intermédiaire de & à - points d'entrée.

Les sorties de chaque panneau (13) peuvent être réalisées par l'intermédiaire de 1 à 6 points de sortie.

### EXEMPLE SELON L'INVENTION

Une unité d'adsorption en lit mobile simulé (ou adsorbeur) de 10 mètres de diamètre est divisé en 6 panneaux méridiens de section équivalente, et alimenté suivant le principe de l'invention présenté sur la figure 1.

Chaque lit a une hauteur de 0,77 mètre.

Le canal de distribution (4) a une hauteur de 19 cm au point le plus haut, c'est-à-dire en entrée des fluides (14). La hauteur du canal décroit ensuite de façon linéaire avec la distance à la paroi d'entrée (14). Le canal de collecte (8) est strictement symétrique au canal de distribution (4). Il a une hauteur croissante depuis le côté gauche jusqu' au côté droit du panneau sur la figure 1.

Des simulations réalisées à l'aide du logiciel de mécanique des fluides numérique FLUENT18.0 montrent que le principe de la compensation des temps de séjour entre la zone d'entrée (3) et la zone de sortie (13) fonctionne correctement. Ce fonctionnement satisfaisant est illustré par la figure 4.

La figure 4 est une visualisation résultant de cette simulation numérique. Il s'agit d'une vue en coupe d'un panneau, selon un plan de coupe de la figure 1. L'entrée (14) dans le canal de distribution se fait par le bord en haut à gauche. Le lit d'adsorbant (6) est la zone comprise entre les deux grilles (5) et (7) schématisées par une ligne pointillée. La sortie du canal (13) de collecte se fait par le bord en bas à droite.

La figure 4 montre une cartographie des temps de séjour (ou âge moyen interne, c'est-à-dire temps s'étant écoulé depuis l'entrée jusqu'à la sortie d'une particule de fluide bien repérée) en tout point du système global comprenant le canal de distribution, le lit d'adsorbant et le canal de collecte.

La grille de niveau de gris représentée en bas sur la figure indique les variations du temps de séjour global entre 0 seconde représenté par un noir et environ 28,3 secondes représenté par un blanc. Ainsi une particule fluide qui vient d'entrer dans le canal de distribution (4) au point (14) a un temps de séjour proche de 0 seconde, et le début du canal de collecte est représenté en noir puis gris foncé. A l'inverse, quand une particule fluide quitte le canal de collecte (7) en bas à droite au point (13), son temps de séjour est de l'ordre de 28,3 secondes, le bout du canal de collecte étant représenté en gris très clair, blanc.

Les iso-lignes de temps de séjour ne sont pas horizontales dans le lit. Sur une même côte verticale au sein du lit, la particule fluide qui est rentrée à gauche dans le lit, près de l'entrée dans la colonne a un temps de séjour très court dans la zone de distribution, donc un temps de séjour global toujours plus élevé que la particule qui est rentrée dans le lit à droite qui a un temps de séjour dans le canal de distribution plus long. En revanche, le temps de séjour dans le lit de toutes les particules fluide est identique.

Les simulations montrent qu'en sortie on a récupéré les écarts de temps de séjour générés dans le canal de distribution (4) en les compensant par les variations de temps de séjour dans le canal de collecte (8). Le profil de temps de séjour se retrouve quasiment perpendiculaire au sens d'écoulement dans le canal de sortie comme dans le canal d'entrée.

Les calculs montrent une dispersion très faible de l'ordre de 2 s² ce qui équivaut à une hauteur équivalente à un plateau théorique de l'ordre de 2 mm. Il s'agit là d'un excellent résultat en terme d'uniformité du temps de séjour.

Dans l'article Augier et al, 2008, on trouve des HETP (hauteur de plateau théorique) typique d'une technologie d'une unité d'adsorption de l'ordre du centimètre.

On peut se reporter à la figure 9 p 473 de l'article cité qui représente la HETP pour différentes vitesses superficielles liquide au sein du lit. On retient la courbe qui correspond à deux configurations de technologie différentes en l'absence d'adsorption. Les valeurs estimées sont comprises entre 12 et 20 cm.

L'invention présente donc un gain d'un rapport 5 à 10 sur la dispersion imputable à l'hydrodynamique dont l'homme du métier sait qu'elle se répercute directement sur les performances du procédé.

## Revendications

1. Dispositif de distribution et de collecte pour les unités de séparation en lit mobile simulé de diamètre supérieur à 4 mètres, l'unité comportant au moins une colonne divisée en N lits d'adsorbant, chaque lit étant divisé en panneaux méridiens, c'est-à-dire en panneaux parallèles entre eux et contigus de manière à assurer une couverture complète de la section dudit lit, le dispositif de distribution et de collecte comprenant des canaux de distribution (4) et des canaux de collecte (8), et chaque panneau étant alimenté par un canal de distribution (4), et le soutirage des effluents dudit panneau se faisant par un canal de collecte (8), **caractérisé en ce que** le canal de distribution (4) et le canal de collecte (8) entourant chaque panneau ont des hauteurs variant linéairement sur toute la longueur du panneau, et qui sont telles que la vitesse d'entrée dans le lit de chaque tranche de fluide reste la même depuis la section d'entrée du panneau jusqu'à sa section de sortie du panneau, et la somme des hauteurs du canal de distribution et du canal de collecte prises en tout point de la longueur du panneau reste constante.

2. Dispositif de distribution et de collecte pour les unités de séparation en lit mobile simulé selon la revendication 1, comprenant des conduites périphériques extérieures, dans lequel le passage des fluides du plateau N au plateau suivant N+1 se fait au moyen d'une conduite périphérique extérieure à la colonne et permettant de relier les différents canaux de collecte du plateau N aux différents plateaux de distribution du plateau N+1, ladite conduite permettant d'effectuer les injections de charge et de solvant et les soutirages d'extrait et de raffinat.

3. Dispositif de distribution et de collecte pour les unités de séparation en lit mobile simulé selon la revendication 2, dans lequel la distribution des fluides sur les différents panneaux se fait par divisions successives en deux du flux provenant de la conduite externe périphérique (10) pour alimenter un groupe de deux entrées (14) de panneaux adjacents, et la collecte des effluents du lit N se fait également par regroupement successifs par deux des sorties (13) de deux panneaux adjacents, qui alimente la conduite périphérique externe (10) du lit N+1.

4. Dispositif de distribution et de collecte pour les unités de séparation en lit mobile simulé selon la revendication 1, comprenant des clarinettes de distribution (15) et des clarinettes de collecte (16), dans lequel la distribution des fluides sur les différents panneaux se fait à partir d'une clarinette de distribution (15) qui alimente directement les différentes entrées (14) de chaque panneau, et la collecte des effluents du lit N se fait de la même manière directement au moyen d'une clarinette de collecte (16) qui récupère les effluents des sorties (13) de chaque panneau.

5. Procédé utilisant le dispositif de distribution et de collecte selon l'une des revendications 1 à 4, dans lequel le fluide entrant est introduit dans chaque panneau du plateau N par l'intermédiaire de conduites d'entrée (14), chaque entrée (14) alimentant un canal de distribution (4) dont la hauteur est maximale en entrée et minimale en sortie dudit canal, chaque tranche de fluide alimentant une fraction du lit granulaire situé immédiatement au-dessous du canal de distribution, et ladite fraction de fluide quittant le lit granulaire pour entrer dans le canal de collecte (8) situé immédiatement au-dessous du lit granulaire, le dit canal de collecte ayant sa hauteur maximale en sortie, l'effluent de sortie étant alors récupéré par la conduite périphérique extérieure (10), et étant réintroduit par les entrées (14) du lit N+1 dans le canal de distribution dudit lit N+1, ledit procédé étant **caractérisé par le fait que** le temps de séjour de chaque tranche de fluide pris depuis l'entrée du plateau N jusqu'à la sortie dudit plateau N, est le même pour chaque tranche de fluide, et la conduite périphérique externe (10) n'apportant pas de dispersion à ce temps de séjour.

6. Application du dispositif de distribution et de collecte selon la revendication 1 au procédé de séparation des xylènes en lit mobile simulé fonctionnant avec un nombre de lits compris entre 4 et 24 et préférentiellement compris entre 8 et 12.

## Patentansprüche

1. Verteilungs- und Sammelvorrichtung für Trennungseinheiten im simulierten Wanderbett mit einem Durchmesser über 4 Meter, wobei die Einheit mindestens eine Säule aufweist, die in N Betten mit Adsorptionsmittel unterteilt ist, wobei jedes Bett in Meridianpaneele unterteilt ist, das heißt in Paneele, die parallel zueinander verlaufen und aneinandergrenzen, um eine vollständige Abdeckung des Abschnitts des Betts sicherzustellen, wobei die Verteilungs- und Sammelvorrichtung Verteilungskanäle (4) und Sammelkanäle (8) umfasst und jedes Paneel von einem Verteilungskanal (4) versorgt wird und die Entnahme der Ausströmungen aus dem Paneel über einen Sammelkanal (8) erfolgt, **dadurch gekennzeichnet, dass** der Verteilungskanal (4) und der Sammelkanal (8), die jedes Paneel umgeben, Höhen aufweisen, die über die gesamte Länge des Paneels linear variieren, und die so beschaffen sind, dass die Eintrittsgeschwindigkeit jeder Fluid-Tranche in das Bett vom Eingangsabschnitt des Paneels bis zu ihrem Ausgangsabschnitt des Paneels dieselbe bleibt, und die Summe der Höhen des Verteilungskanals und des Sammelkanals, die an jedem Punkt der Länge des Paneels gemessen werden, konstant bleibt.

2. Verteilungs- und Sammelvorrichtung für Trennungseinheiten im simulierten Wanderbett nach Anspruch 1, umfassend äußere periphere Leitungen, wobei der Übergang der Fluide von der Platte N zur nächsten Platte N+1 mittels einer peripheren Leitung erfolgt, die sich außerhalb der Säule befindet und es gestattet, die verschiedenen Sammelkanäle der Platte N mit den verschiedenen Verteilungsplatten der Platte N+1 zu verbinden, wobei es die Leitung gestattet, die Füllstoff- und Lösungsmitteleinspritzungen sowie die Entnahmen von Extrakt und Raffinat vorzunehmen.

3. Verteilungs- und Sammelvorrichtung für Trennungseinheiten im simulierten Wanderbett nach Anspruch 2, wobei die Verteilung der Fluide auf die verschiedenen Platten durch aufeinanderfolgende Teilungen in zwei des Flusses von der äußeren peripheren Leitung (10) erfolgt, um eine Gruppe von zwei Eingängen (14) benachbarter Paneele zu versorgen, und die Sammlung der Ausströmungen aus dem Bett N ebenfalls durch aufeinanderfolgende Zweiergruppierung der Ausgänge (13) zweier benachbarter Paneele, die die äußere periphere Leitung (10) des Betts N+1 versorgt, erfolgt.

4. Verteilungs- und Sammelvorrichtung für Trennungseinheiten im simulierten Wanderbett nach Anspruch 1, umfassend Verteilungsklarinetten (15) und Sammelklarinetten (16), wobei die Verteilung der Fluide auf die verschiedenen Platten ausgehend von einer Verteilungsklarinette (15) erfolgt, die die verschiedenen Eingänge (14) jeder Platte direkt versorgt, und die Sammlung der Ausströmungen aus dem Bett N auf dieselbe Weise direkt mittels einer Sammelklarinette (16) erfolgt, die die Ausströmungen aus den Ausgängen (13) jeder Platte auffängt.

5. Verfahren unter Verwendung der Verteilungs- und Sammelvorrichtung nach einem der Ansprüche 1 bis 4, wobei das eintretende Fluid in jedes Paneel der Platte N mittels Eingangsleitungen (14) eingeleitet wird, wobei jeder Eingang (14) einen Verteilungskanal (4) versorgt, dessen Höhe am Eingang maximal und am Ausgang des Kanals minimal ist, wobei jede Fluid-Tranche einen Anteil des Granulatbetts versorgt, das sich unmittelbar unter dem Verteilungskanal befindet, und wobei der Fluidanteil das Granulatbett verlässt, um in den Sammelkanal (8) einzutreten, der sich unmittelbar unter dem Granulatbett befindet, wobei der Sammelkanal seine maximale Höhe am Ausgang aufweist, wobei die Ausgangsausströmung dann von der äußeren peripheren Leitung (10) aufgefangen und durch die Eingänge (14) des Betts N+1 wieder in den Verteilungskanal des Betts N+1 eingeleitet wird, wobei das Verfahren **dadurch gekennzeichnet ist, dass** die Verweilzeit jeder Fluid-Tranche, die vom Eingang der Platte N bis zum Ausgang der Platte N gemessen wird, für jede Fluid-Tranche dieselbe ist, und die äußere periphere Leitung (10) dieser Verweilzeit keine Streuung bringt.

6. Anwendung der Verteilungs- und Sammelvorrichtung nach Anspruch 1 auf das Verfahren zur Trennung der Xylole im simulierten Wanderbett, das mit einer Anzahl von Betten zwischen 4 und 24 und vorzugsweise zwischen 8 und 12 funktioniert.

## Claims

1. Distribution and collection device for simulated moving bed separation units with a diameter of greater than 4 meters, the unit comprising at least one column divided into N beds of adsorbent, each bed being divided into meridian panels, i.e. into panels that are mutually parallel and contiguous so as to ensure complete coverage of the cross section of said bed, the distribution and collection device comprising distribution channels (4) and collection channels (8), and each panel being fed by a distribution channel (4), and the withdrawal of the effluents from said panel taking place via a collection channel (8), **characterized in that** the distribution channel (4) and the collection channel (8) surrounding each panel have heights that vary linearly over the entire length of the panel, and which are such that the inlet velocity into the bed of each portion of fluid remains the same from the inlet section of the panel to its outlet section of the panel, and the sum of the heights of the distribution channel and of the collection channel taken at any point of the length of the panel remains constant.

2. Distribution and collection device for simulated moving bed separation units according to Claim 1, comprising outer peripheral ducts, wherein the passage of the fluids from plate N to the next plate N+1 takes place by means of a peripheral duct outside of the column and that makes it possible to connect the various collection channels of the plate N to the various distribution plates of the plate N+1, said duct making it possible to carry out injections of feedstock and of solvent and withdrawals of extract and of raffinate.

3. Distribution and collection device for simulated moving bed separation units according to Claim 2, wherein the distribution of the fluids over the various panels takes place by successive divisions into two of the stream originating from the outer peripheral duct (10) in order to supply a group of two inlets (14) of adjacent panels, and the collection of the effluents from the bed N also takes place by successive combining, in twos, of the outlets (13) of two adjacent panels, which feeds the outer peripheral duct (10) of the bed N+1.

4. Distribution and collection device for simulated moving bed separation units according to Claim 1, comprising distribution manifolds (15) and collection manifolds (16), wherein the distribution of the fluids over the various panels takes place from a distribution manifold (15) which directly supplies the various inlets (14) of each panel, and the collection of the effluents from the bed N takes place in the same manner directly by means of a collection manifold (16) which recovers the effluents from the outlets (13) of each panel.

5. Process using the distribution and collection device according to one of Claims 1 to 4, wherein the incoming fluid is introduced into each panel of the plate N by means of inlet ducts (14), each inlet (14) supplying a distribution channel (4), the height of which is maximum at the inlet and minimum at the outlet of said channel, each portion of fluid supplying a fraction of the granular bed located immediately below the distribution channel, and said fraction of fluid leaving the granular bed in order to enter into the collection channel (8) located immediately below the granular bed, said collection channel having its maximum height at the outlet, the outlet effluent then being recovered by the outer peripheral duct (10), and being reintroduced via the inlets (14) of the bed N+1 into the distribution channel of said bed N+1, said process being **characterized in that** the residence time of each portion of fluid taken from the inlet of the plate N to the outlet of said plate N, is the same for each portion of fluid, and the outer peripheral duct (10) not adding any dispersion to this residence time.

6. Application of the distribution and collection device according to Claim 1 to the process for separating xylenes in a simulated moving bed operating with a number of beds of between 4 and 24, and preferentially of between 8 and 12.
